# EUROPÄISCHE PATENTANMELDUNG

(11) **EP 2 133 044 A2**
(43) Veröffentlichungstag der Anmeldung: **16.12.2009**
(21) Anmeldenummer: 09160146.8
(22) Anmeldetag: 13.05.2009
(51) Int. Cl.: A61F 2/86, A61F 2/92

(54) **Wirkstoffbeladenes Implantat**

(30) Priorität: 12.06.2008 DE 102008002395
(71) Anmelder: Biotronik VI Patent AG, 6341 Baar (CH)
(72) Erfinder: Borck, Alexander, 91086, Aurachtal (DE); Diener, Tobias, 91058, Erlangen (DE); Harder, Claus, 91080, Uttenreuth (DE); Tittelbach, Michael, 90429, Nürnberg (DE); Fargahi, Amir, 5242, Birr (CH)
(74) Vertreter: Lindner-Vogt, Karin L.

(57) **Zusammenfassung**

Die Erfindung betrifft ein wirkstoffbeladenes Implantat (100) mit einem Trägerkörper (10) und wenigstens einem Wirkstoff (50) zur Freisetzung in einem Freisetzungsgebiet. Der Wirkstoff (50) ist zur Krebstherapie und/oder palliativen Behandlung vorgesehen und ist im bestimmungsgemäßen Wirkzustand des Implantats (100) in einem Körper eines Lebewesens lokal und/oder regional gesteuert mit vorgebbarer Rate und/oder über eine vorgebbare Zeitdauer freisetzbar.

## Beschreibung

Die Erfindung betrifft ein wirkstoffbeladenes Implantat nach dem Oberbegriff des Patentanspruchs 1.

Im Bereich der lokalen bzw. regionalen Therapie ist es notwendig, einen oder mehrere Wirkstoffe in hoher Dosierung zu verabreichen, ohne dabei durch Wirkungsweise und/oder Dosierungshöhe des Medikaments negative Nebenwirkungen in umliegendem Gewebe oder Körperregionen außerhalb des Zielgebietes zu induzieren.

Die medikamentelle Therapie und/oder palliative Behandlung kanzerogenen Gewebes erfordert hochdosierte Gaben z.B. zytostatischer Pharmaka und/oder Opiate. Die bei oraler oder intravenöser Gabe verbundenen Nebenwirkungen auf gesunde Gewebsregionen oder Organe sind hierbei nicht unerheblich und Tragen zu einer erhöhten Morbidität der Patienten bei.

Bei inoperablen Tumorerkrankungen, insbesondere einem HCC (HepatoCellular Carcinoma) werden unterschiedliche Therapien angewandt, die alle mit großen Nachteilen verbunden sind. Bekannte Beispiele sind transarterielle Tumorembolisation (TACE), arterielle Chemoperfusion (HAI), Kryotherapie, laserinduzierte Thermotherapie (LITT), Radiofrequenz-Ablation (RFA), perkutane Ethanolinjektion (PEI). Insbesondere bei der TACE könne große Teile von gesundem Lebergewebe geschädigt werden, was in einer fehlenden hepatischen Funktionsreserve resultiert. Die Therapien werden angewandt, um die Wartezeit bis zur Lebertransplantation zu überbrücken. Ein kurativer Effekt ist nicht vorhanden.

Die Gabe von Einzeldosen von Medikamenten ist oft unzureichend dosiert, zudem sinkt die Wirkstoffkonzentration häufig zu schnell unter das therapeutische Fenster ab. Die systemische Gabe von Zytostatika ist bei Lebertumoren auch aufgrund des Allgemeinzustandes der Patienten nicht durchführbar, weshalb es zu Nachbehandlungen und längerem Klinikaufenthalt für den Patienten kommt.

Aus der Offenlegungsschrift US 2002/0133224 A1 ist ein Stent bekannt, der von einer mikroporösen Polymermembran umgeben ist, in die ein pharmazeutischer Wirkstoff eingebettet sein kann. Das Patent US 7,056,339 B2 offenbart einen Stent, mit einer wirkstoffbeladenen Matrix in abluminalen und adluminalen Kanälen an der Oberfläche des Stents. Der Wirkstoff ist in Mikrosphären enthalten. Die Außenseite des Stents ist mit einem kovalent gebundenen Gel umgeben. Der Wirkstoff kann über eine längere Zeit abgegeben werden.

Der Erfindung liegt die Aufgabe zugrunde, ein wirkstoffbeladenes Implantat zu schaffen, das einen Wirkstoffe in hoher Dosierung bereitstellt, der durch Art und/oder Wirksamkeit bei systemischer Gabe nicht oder nicht in vergleichbarer Potenz einsetzbar ist.

Die Aufgabe wird erfindungsgemäß durch die Merkmale der Patentanspruchs 1 gelöst. Günstige Ausgestaltungen und Vorteile der Erfindung ergeben sich aus den weiteren Ansprüchen und der Beschreibung.

Es wird ein wirkstoffbeladenes Implantat mit einem Trägerkörper und wenigstens einem Wirkstoff zur Freisetzung in einem Freisetzungsgebiet vorgeschlagen, bei dem der Wirkstoff zur Krebstherapie und/oder palliativen Behandlung vorgesehen ist und im bestimmungsgemäßen Wirkzustand des Implantats in einem Körper eines Lebewesens lokal und/oder regional gesteuert mit vorgebbarer Rate und/oder über eine vorgebbare Zeitdauer freisetzbar ist. Das Implantat kann, je nach Ausgestaltung, zum Einsetzen in ein Blutgefäß oder zum Einsetzen in ein Gewebe des Körpers vorgesehen sein. Es ist vorteilhaft möglich, den oder die Wirkstoffe gezielt in hoher Dosierung lokal zu verabreichen, ohne dabei durch Wirkungsweise und/oder Dosierungshöhe des Medikaments negative Nebenwirkungen in umliegendem Gewebe oder in umliegenden Körperregionen zu induzieren. Ferner ist es möglich, ein Implantat mit sehr hohem Wirkstoffgehalt zu schaffen. In der Regel ist ein hoher Wirkstoffgehalt bei bekannten Implantaten der mechanischen Stabilität sehr abträglich.

"Wirkstoff" bezeichnet im vorliegenden Kontext generell einen einzelnen Wirkstoff, eine Mischung aus Wirkstoffen oder eine Wirkstoffformulierung und/oder ein anderes Material, das gezielt zur Freisetzung vorgesehen ist, vorteilhaft mit pharmazeutischer bzw. biologischer Wirksamkeit. Vorteilhaft kann das wirkstoffbeladene Implantat mit Zytostatika beladen sein, die bei systemischer Gabe, z.B. mittels oraler Aufnahme, durch ihre Art bzw. ihre Wirksamkeit zu schweren Nebenwirkungen führen würden. Die lokale Gabe des Wirkstoffs macht es möglich, Dosierungen an einen Wirkungsort zu bringen, der systemisch nicht mit vergleichbarer Potenz erreichbar ist. So kann das Implantat z.B. in einem Blutgefäß einige Zentimeter vor einem Tumor eingebracht werden, wobei der Wirkstoff vom Blutstrom zum Tumor transportiert wird. Zusätzlich kann die Wirkstoffabgabe gesteuert ablaufen, so dass der Wirkstoff über eine ausreichend lange Zeit zur Verfügung gestellt wird.

Vorteilhaft kann die Freisetzung des Wirkstoffs sich an den gewünschten Eckwerten orientieren. So kann eine möglichst homogene Freisetzung des Wirkstoffs über einen längeren Zeitraum, z.B. zwei Wochen oder mehr erreicht werden. Ein Dosis-Peak kann relativ kurzfristig, etwa nach spätestens vierundzwanzig Stunden, erreicht sein. Der Dosisabfall nach Ende des Behandlungszeitraums kann geeignet eingestellt werden; bevorzugt wird dieser möglichst steil eingestellt. Die Freisetzung kann, je nach Ausgestaltung des Implantats, durch Elution oder durch Diffusion erfolgen. Vorstellbar ist auch, systemisch einen gewünschten, relativ niedrigen Wirkstoffspiegel im Blut einzustellen, der von einem kurzfristigen Peak des erfindungsgemäßen Implantats überlagert ist.

Besonders vorteilhaft ist, wenn der Trägerkörper biodegradierbar ausgebildet sein kann. Dann kann eine Entfernung des Trägerkörpers nach Ende des Behandlungszeitraums unterbleiben. Vorteilhaft kann der Trägerkörper aus einem Material gebildet sein, das hinreichend langsam abgebaut wird, insbesondere wesentlich langsamer, als die Freisetzung des Wirkstoffes beansprucht. Es können auch Trägerkörper verwendet werden, die z.B. Embolisationspartikel als Wirkstoff freisetzen und die z.B. im Inneren ein oder mehrere Medikamente zur Freisetzung mit sich tragen.

Gemäß einer bevorzugten Ausgestaltung kann der Wirkstoff in eine Polymermatrix eingebettet sein, aus der der Wirkstoff durch Degradation der Polymermatrix und/oder durch Diffusion freisetzbar ist. Die Polymermatrix kann als Beschichtung auf dem Trägerkörper vorgesehen sein. Ist der Trägerkörper z.B. ein Stent, können die einzelnen Streben des Stents von der Polymermatrix umgeben sein, während Zwischenräume des Stents offen sind. Vorteilhaft kann der Trägerkörper wenigstens bereichsweise mit der Polymermatrix beschichtet sein.

Alternativ kann die Polymermatrix in der Art einer Membran oder Hülle den Trägerkörper umgeben. Ist der Trägerkörper z.B. ein Stent, sind sowohl die Streben als auch die Zwischenräume zwischen den Streben des Stents außenumfänglich abluminal und/oder innenumfänglich (ad)luminal von der Polymermatrix überdeckt. Eine zeitlich variable Freisetzung kann durch die Verwendung von degradierbaren bzw. resorbierbaren Polymeren mit unterschiedlichen Degradationsgeschwindigkeiten ermöglicht werden.

Günstigerweise kann der Wirkstoff in Vertiefungen und/oder Hohlräumen des Trägerkörpers angeordnet sein. Es kann eine besonders hohe Menge an Wirkstoff deponiert werden. Bevorzugt kann das Freisetzen des Wirkstoffs durch Elution oder Diffusion erfolgen. Es ist jedoch alternativ oder zusätzlich auch möglich, Wirkstoff in einer Polymermatrix in den Vertiefungen und/oder Hohlräumen anzuordnen. In diesem Fall kann das Freisetzen des Wirkstoffs verzögert erfolgen. Eine zeitlich variable Freisetzung kann durch die Verwendung von degradierbaren bzw. resorbierbaren Polymeren mit unterschiedlichen Degradationsgeschwindigkeiten ermöglicht werden.

Vorteilhaft kann die Polymermatrix wenigstens bereichsweise den Trägerkörper bilden. Eine derartige selbstragende Polymermatrix kann z.B. als Schlauch oder Stift ausgebildet sein.

Der Wirkstoff kann vorteilhafterweise zwischen zwei Polymerschichten angeordnet sein, welche den hohlen Trägerkörper adlumal und ablumal umgeben. Vorteilhaft kann die Freisetzung des Wirkstoffs z.B. durch Elution oder Diffusion erfolgen. Durch die Vorgabe von Kanalgeometrien z.B. in oder zwischen den Polymerschichten kann die Abgaberate leicht eingestellt werden.

Alternativ kann der Trägerkörper durch einen Schlauch gebildet sein, der bereichsweise umgestülpt ist, wobei der Wirkstoff im Bereich eines durch die Umstülpung gebildeten Hohlraums zwischen dem inneren Schlauchabschnitt und dem darüber liegenden, umgestülpten Schlauchabschnitt angeordnet ist. Der Schlauch ermöglicht eine einfache Herstellung mit guten mechanischen Eigenschaften.

Zur Behandlung spezieller Erkrankungen der Leber können günstigerweise in einer Polymermatrix freizusetzende Embolisationspartikel vorgesehen sein, welche vor oder nach einer vorzugsweise lokalen oder regionalen Wirkstoffgabe ein das Implantat enthaltendes Blutgefäß gezielt verschließt. Bevorzugt können die Polymermatrix und/oder die Embolisationspartikel mit Wirkstoff beladen sein.

Es kann eine wirkstoffundurchlässige Deckschicht auf dem Implantat vorgesehen sein, die im implantierten Zustand das abluminale Gewebe des Freisetzungsgebiets gegen den Wirkstoff schützt. Die wirkstoffundurchlässige Deckschicht kann permanent bzw. nichtresorbierbar ausgebildet sein oder aus resorbierbarem Material bestehen, das eine deutlich geringere Degradationsgeschwindigkeit aufweist als die Freisetzungsgeschwindigkeit des Wirkstoffs. So kann der Wirkstoff sicher in vorgegebener Weise freigesetzt werden, bevor der Trägerkörper abgebaut wird.

Vorteilhaft kann der Trägerkörper als Stent ausgebildet sein, vorzugsweise als selbstexpandierender Stent. Der Trägerkörper kann alternativ als Rohr oder Stab als so genannter Wirkstoffstift ausgebildet sein. Als Rohr kann der Trägerkörper vorzugsweise ein Hohlrohr sein, wobei die Rohrwand mit Wirkstoff beladen ist. Dieser kann z.B. in einer Polymermatrix eingebettet sein, welche auf der Rohrwand angeordnet ist, oder kann in Hohlräumen oder Vertiefungen der Rohrwand angeordnet sein, die dann entsprechend z.B. porös oder aufgeraut ausgebildet sein kann. Als Stab weist der Trägerkörper vorzugsweise einen wirkstoffhaltigen Kern auf, aus dem der Wirkstoff durch eine geeignet durchlässige Stabwand und/oder aus einer oder beiden Stirnseiten des Kerns austreten kann. Eine solche Ausgestaltung in Form eines Wirkstoffstifts ist vorteilhaft für ein Einbringen ins Gewebe. Es ergibt sich jeweils eine relativ stabile und belastbare Vorrichtung.

Wenn das erfindungsgemäße Implantat zum Einbringen in ein Blutgefäß vorgesehen ist und an oder in der Gefäßwand fixierbar ist, muss es keine so große Stützkraft aufweisen wie etwa Stents. Es kann dann auf Kosten der Stützkraft das Design vorteilhaft so ausgelegt werden, dass mehr Wirkstoff gespeichert werden kann.

Gemäß einer weiteren vorteilhaften Möglichkeit kann der Trägerkörper aus einer gerollten Folie gebildet sein, die Vertiefungen zur Aufnahme des Wirkstoffs aufweist. Zusätzlich oder alternativ kann der Trägerkörper aus einer gerollten und geschlitzten Folie gebildet sein. Die Folie kann aus einem permanenten Material, vorzugsweise einer Nickel-Titan-Legierung (Nitinol), oder auch aus einem biodegradierbaren Material gebildet sein.

Die Erfindung ist nachfolgend beispielhaft, anhand von in Zeichnungen dargestellten Ausführungsbeispielen, näher erläutert. Es zeigen in schematischer Darstellung:
- Fig. 1: einen Querschnitt eines bevorzugten Implantats nach einer bevorzugten ersten Ausgestaltung der Erfindung;
- Fig. 2a-2d: verschiedene Varianten eines bevorzugten Implantats in Form eines Wirkstoffstifts nach einer Ausgestaltung der Erfindung zum Einbringen bevorzugt in ein Gewebe;
- Fig. 3: ein bevorzugtes Einführsystem für ein bevorzugtes Implantat nach der Erfindung gemäß Fig. 2a-2d;
- Fig. 4a-4c: verschiedene Ansichten eines bevorzugten Implantats nach einer weiteren bevorzugten Ausgestaltung der Erfindung mit einem Trägerkörper, der zwischen zwei Polymerschichten angeordnet ist;
- Fig. 5a, 5b: eine wirkstoffbeladene Polymermatrix (Fig. 5a) und ein Trägerkörper mit der wirkstoffbeladenen Polymermatrix (Fig. 5b) nach einer weiteren bevorzugten Ausgestaltung der Erfindung;
- Fig. 6: eine Ansicht eines aus einer Polymermatrix gebildeten Trägerkörpers nach einer weiteren Ausgestaltung der Erfindung;
- Fig. 7a, 7b: einen Längsschnitt durch ein bevorzugtes Implantat nach einer weiteren bevorzugten Ausgestaltung der Erfindung (Fig. 7a) und einen Querschnitt durch eine Variante des Implantats (Fig. 7b);
- Fig. 8a, 8b: einen bevorzugten Trägerkörper (Fig. 8a) und eine Einbausituation des Trägerkörpers (Fig. 8b); und
- Fig. 9a, 9b: einen bevorzugten Trägerkörper im gerollten Zustand (Fig. 9a) und im ausgerollten Zustand (Fig. 9b) nach einer weiteren Ausgestaltung der Erfindung.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

Ein erfindungsgemäßes wirkstoffbeladenes Implantat umfasst einen Trägerkörper und einem Wirkstoff zur Freisetzung in einem Freisetzungsgebiet, wobei der Wirkstoff zur Krebstherapie und/oder palliativen Behandlung vorgesehen ist und im bestimmungsgemäßen Wirkzustand des Implantats in einem Körper eines Lebewesens lokal und/oder regional gesteuert mit vorgebbarer Rate und/oder über eine vorgebbare Zeitdauer freisetzbar ist. Je nach Ausführung kann der Trägerkörper dazu vorgesehen sein, in ein Blutgefäß oder ins Gewebe eingesetzt zu werden. Der Trägerkörper kann mit einem Polymer beschichtet oder bedeckt sein, oder aus einem Polymer bestehen, das biologisch degradierbar ist und sich im Körper auflöst. Gegebenenfalls kann das Implantat auch ein nicht degradierbares Material umfassen.

Bevorzugte Wirkstoffe zur Anwendung in reiner Form, oder inkorporiert in einer Polymermatrix, sind insbesondere Medikamente, die speziell zur Krebstherapie oder palliativen Behandlung von Krebs geeignet sind. Zudem kann eines oder mehrere Medikamente ausgewählt werden aus den Gruppen der (einige der folgenden Bezeichnungen können Markennamen sein):
Immunsuppressiva (z.B. Sirolimus),
Calcineurininhibitoren (z.B. Tacrolimus),
Antiflogistika (z.B. Cortison, Dichlofenac),
Antünflammatorica (z.B. Imidazole, Pimecrolimus),
Steroide,
Proteine / Peptide,
insbesondere eines oder mehrere Medikamente zur Krebstherapie oder palliativen Behandlung von Krebs wie z.B.:
   - 105AD7
   - 13-cis RA
   - 17-AAG
   - 1A7
   - 2A11
   - 3F8
   - 3H1
   - 5-Fluorouracil
   - 9-cis-retinoic acid
   - A10/AS2-1
   - ABT-51 0
   - ABX-EGF
   - Ad p53
   - Adriamycin PFS
   - Adriamycin RDF
   - Alemtuzumab
   - Alitretinoin
   - Allovectin-7
   - Altretamine
   - Amifostine
   - Angiostatin
   - Angiozyme
   - Antineoplastons
   - Anti-Tac-PE38 (LMB-2)
   - AP12009
   - Aplidine
   - Apomine
   - Aromasin
   - Arsentrioxid
   - Astrasentan
   - Azathioprine
   - Bay 43-9006
   - Bay 50-4798
   - Bay 12-9566
   - BB-10010
   - BB-10901
   - BEC2
   - Bevacizumab
   - Bexarotene
   - Bicalutamide
   - BL22
   - BMS-214662
   - BMS-247550
   - BMS-275291
   - BNP7787
   - Bryostatin-1
   - Busulfan
   - Busulfex
   - Buthionine sulphoximine
   - Capecitabine
   - Carboxyamidotriazole
   - Casodex
   - CC-5013
   - CCI-779
   - Celecoxib
   - Cetuximab (Erbitux)
   - Ch14.18
   - CHS828
   - CI-1040
   - CI-994
   - Cisplatin
   - Clodronate
   - CM101
   - COL-3
   - Combretastatin A4
   - CP-461
   - CP-471,358
   - CP-547,632
   - CT 2584
   - Cyclophosphamide
   - Cyclosporin A
   - Cytoxan
   - Decitabine
   - Denileukin diftitox (ONTAK)
   - Depsipeptide
   - Dexniguldipin
   - Dexrazoxane
   - Dexverapamil
   - Dolastatin-10
   - Doxorubicin
   - DPPE
   - EMD 273063
   - EMD 55900
   - EMD 72000
   - Endostatin
   - Enzyme L-asparaginase
   - EP0906
   - Epirubicin
   - Epratuzumab
   - ET-743
   - Exemestane
   - Exisulind
   - FB642
   - Femara
   - Fenretinide
   - Finasteride
   - FK317
   - FK866
   - Flavopiridol
   - G17DT
   - GBC-590
   - GD0039
   - GEM231
   - Gemtuzumabozogamicin
   - Genasense (Fa.Genta)
   - GF120918
   - GM-CSF
   - GW572016
   - H22xKI-4
   - Hexalen
   - HSV-TK VPC
   - HuM195
   - HuMV833
   - ICR62
   - IL13-PE38QQR
   - IL-2/histamine
   - Ilmofosine
   - ILX23-7553
   - IM862
   - IMC-1C11
   - Imuran
   - ING-1
   - Interleukin-12
   - INX3280
   - Irofulven
   - ISIS-2503
   - ISIS-3521
   - ISIS-5132
   - J591
   - Kahalalide F
   - KM871
   - KW-2189
   - L-778 123
   - LAF389
   - LAK, TIL, CTL
   - LErafAON
   - Letrozole
   - Lobradimil
   - Lovastatin
   - LU103793
   - LY-293111
   - LY-317615
   - LY-335979
   - LY-355703
   - Lyprinol
   - Marimastat
   - MCC-465
   - MDX-010
   - MDX-11
   - MDX-447
   - MDX-H210
   - Melatonin
   - Methotrexate
   - MG98
   - Mifeprex
   - Mifepristone
   - Mitoxantrone
   - MMI270
   - MS209
   - Myleran
   - Mylotarg
   - Mytomicin C
   - Natalizumab
   - Neosar
   - Neovastat
   - Nolvadex
   - NV1020
   - Oblimersen (Genasense)
   - OK-432
   - OL(1)p53
   - Oregovomab
   - OSI-774 (Tarceva)
   - p53
   - Panretin
   - Perifosine
   - Phenoxodiol
   - Phenylacetate
   - Phenylbutyrate
   - PI-88
   - Pioglitazone
   - Pivaloyloxy-methylbutyrate
   - PKC 412
   - PKI 166
   - PNU-145156E
   - PNU-166196
   - Prinomastat
   - Propecia
   - Proscar
   - PS-341
   - PSC 833
   - PSK
   - PTK/ZK 787
   - PV701
   - Pyrazoloacridine
   - Quinine
   - R-101933
   - R115777 (Zarnestra)
   - Reolysin
   - RhuMab-VEGF
   - Rituximab
   - RO 31-7453
   - RPR/INGN-201
   - Rubex
   - SB-408075
   - SCH66336
   - SGN-15
   - Squalamine
   - SS1-PE38
   - STI571 (Gleevec)
   - SU-101
   - SU5416
   - SU6668
   - Suramin
   - Swainsonine
   - TAC-101
   - Tamoxifen
   - Taurolidine
   - Tazarotene
   - Temodar
   - Temozolomide
   - tgDCC-E1A
   - Thalidomide
   - Tirapazamine
   - TK gene
   - TLK286
   - TNP-470
   - TP-38
   - Transretinoic acid
   - Trastuzumab (Herceptin)
   - Trelstar Depot
   - TriGem
   - Triptorelin
   - Troglitazone
   - Ubenimex
   - UCN-01
   - Vaccines
   - Verapamil
   - Vitxain
   - WX-G250
   - XR9576
   - ZD1839 (Iressa)
   - ZD6126
   - ZD6474
   - E7070
   - Edrecolomab
   - E1A-lipid complex (Fa.Targeted Genetics)
   - GX01 (Fa. Gemin X Biotechnologies)
   - Immunoconjugate Antikörper mit Toxin
   - INGN201 (Fa. Introgen Therapeutics)
   - ONYX-015 (Fa. Onyx Pharmaceuticals)
   - SCH58500 (Fa. Schering-Plough)
   - Suberoylanilidehydroxamic acid
   - TRAIL (Fa. Genentech/Immunex)
   - A5B7 mit Carboxypeptidase A

Geeignete Polymere zur Anwendung bei dem erfindungsgemäßen Implantat sind beispielhaft im Weiteren aufgeführt, speziell unter dem Aspekt unterschiedlicher Resorptions- bzw. Degradationsgeschwindigkeit:
- Langsam resorbierbare / bioresorbierbare / degradierbare Polymere:
   Polydioxanon, Polyglycolid, Polylactide [Poly-L-Lactid, Poly D,L Lactid, und Copolymere sowie Blends wie Poly(L-Lactid-co-glycolid), Poly(D,L-lactid-co-glycolid), Poly(L-Lactid-co-D,L-Lactid), Poly(L-Lactid-co-trimethylen carbonat)], Poly-εcaprolacton, Di- und Triblockcopolymere der oben genannten Lactide mit Polyethylenglycol, Polyhydroxyvalerat, Ethylvinylacetat, Polyethylenoxid, Polyphosphorylcholin, Polyhydroxybuttersäure (ataktisch, isotaktisch, syndiotaktisch sowie deren Blends), Polyorthoesther, Polyanhydride, etc.
- Schnell resobierbare / bioresorbierbare / degradierbare Materialien:
   Fette, Lipide (z.B. Cholesterin, Cholesterinesther und Mischungen daraus), Saccharide (Alginat, Chitosan, Levan, Hyaluronsäure und Uronide, Heparin, Dextran, Nitrocellulose, Celloloseacetat bzw. Derivate der Cellulose, Maltodextrin, Chondroidinsulfat, Carragenan, etc.), Fibrin, Albumin, Polypeptide und deren Derivate etc.

Vorteilhaft ist es, eine vollständige Resorption aller Bestandteile im Organismus zu erreichen. In manchen Fällen, insbesondere bei palliativer Therapie, können auch nichtresorbierbare Materialien zum Einsatz kommen. Bevorzugte Materialien für die Einzelkomponenten sind hierbei für:
- Trägerkörper wie Stent oder Stäbchen, Rohr, Gitter:
   CoCr-Legierungen
   Medizinischer Edelstahl 316L
   Nickel-Titanlegierung
- Beschichtungen und/oder Membranen aus nichtresorbierbaren / permanenten Polymeren:
   Polypropylen, Polyethylen, Polyvinylchlorid, Polyacrylate (Polyetyl- und Polymethylacrylate, Polymethylmetacrylat, Polymethyl-co-ethyl-acrylat, Ethylen/Ethylacrylat etc.), Polytetrafluorethylen (Ethylen/Chlortrifluorethylen Copolymere, Ethylen/Tretrafluorethylen Copolymere), Polyamide (Polyamidimid, Trogamid PA-11, PA-12, PA-46, PA-66 etc.), Polyether Block Amid (Pebax mit verschiedenen Härten), Polyetherimid, Polyethersulfon (und Blends), Polyesther, Polycarbonat, Polyphenylsulfone (und Blends), Poly(iso)butylen, Polyether-etherketon (PEEK) und seine Blends (mit z.B. PES), Polyvinylchlorid, Polyvinylfluorid, Polyvinylalkohol, Polyvinylacetat, Polyurethan (z.B. Pellethane, Elasthane), Polybuthylenterephthalat, Silikone, Polyphosphazen, Polyphenylen, Polymerschäume (aus z.B. Carbonaten, Styrolen etc.), sowie die Copolymere und Blends der aufgezählten Klassen, bzw. der Klasse der Thermoplaste und Elastomere im Allgemeinen.

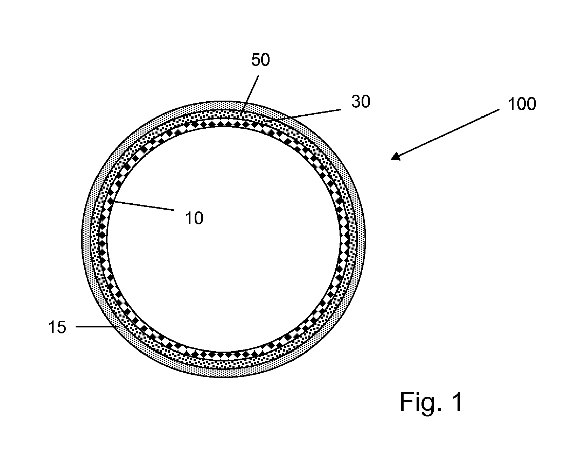

Zur Erläuterung der Erfindung zeigt Fig. 1 einen Querschnitt einer Ausführung eines bevorzugten wirkstoffbeladenen Implantats 100 mit einem Trägerkörper 10 und einem Wirkstoff 50 zur Freisetzung in einem Freisetzungsgebiet. Der Trägerkörper 10 ist hohl ausgebildet und kann z.B. in einem Blutgefäß positioniert werden. Der Wirkstoff 50 kann in den Blutstrom abgegeben und zu seinem Wirkungsort transportiert werden.

Das wirkstofftragende Implantat 100 ist vorzugsweise mit Zytostatika beladen, die bei systemischer Gabe durch ihre Art bzw. ihre Wirksamkeit zu schweren Nebenwirkungen führen würden. Die lokale Gabe macht es möglich, Dosierungen an einen Wirkungsort zu bringen, der systemisch nicht mit vergleichbarer Potenz erreichbar ist. Zusätzlich kann die Wirkstoffabgabe gesteuert ablaufen, so dass der Wirkstoff 50 über eine ausreichend lange Zeit zur Verfügung gestellt wird.

Vorzugsweise kann sich die Elution des Wirkstoffs an den folgenden Eckwerten orientieren:
- Möglichst homogene Freisetzung des Wirkstoffs über mindestens 2 Wochen;
- Dosis-Peak sollte nach spätestens 24 Stunden. erreicht sein;
- der Dosisabfall nach Ende des Behandlungszeitraums sollte möglichst steil sein.

Als bevorzugter Trägerkörper 10 dient z.B. ein aus dem koronaren oder peripheren Bereich bekannter (vorzugsweise selbstexpanderiender) Stentgrundkörper, der mit einem Wirkstoff 50, der in eine Polymermatrix 30 eingebettet ist, beschichtet ist. Der beschichtete Trägerkörper 10 wird dann auf einen Dorn montiert und nur außen mit einer undurchlässigen Beschichtung 15 versehen. Als undurchlässige Beschichtung 15 können Polymere wie Parylen, PES, PTFE und andere zum Einsatz kommen. Die Beschichtung 15 erfolgt, indem das Polymer zusammen mit dem Wirkstoff 50 in Lösung gebracht und über einen Tauchprozess oder eine Sprühbeschichtung auf den Trägerkörper 10 aufgebracht wird. Die Beschichtung 15 schützt im bestimmungsgemäßen Wirkzustand des Implantats in einem Blutgefäß die Gefäßwand vor einer unmittelbaren Einwirkung des Wirkstoffs 50. Der Blutstrom kann den Wirkstoff 50 zum eigentlichen Wirkort transportieren.

Als Material für den Trägerkörper 10 eignen sich grundsätzlich u.a. CoCr, 316L oder Mg, das bevorzugte Material ist jedoch eine Nickel-Titan-Legierung (Nitinol). Als Design für den Trägerkörper 10 wird ein Wallstent zugrunde gelegt, wobei das Implantat 100 vorteilhafterweise selbstexpandierend ist.

Ein Wirkstoff 50 mit zytostatischen Eigenschaften, bevorzugt Doxorubicin, Epirubicin, Cisplatin, Mitomycin C (als Einzelsubstanz oder in Kombination), wird in degradable Polymere wie z.B. poly-L-Lactide, poly-D-Lactide, Polyglycolide, Polydioxanone, Polycaprolactone, und Polygluconate, Polylactic-acid-polyethylene-oxide-Copolymere, modifizierte Cellulose, Collagen, Poly(hydroxybutyrate), Polyanhydride, Polyphosphoester, Poly(amino-Säuren), Poly(alpha-hydroxy-Säure) und/oder Kombinationen eingebracht.
Denkbar sind auch nichtabbaubare Polymere wie:
Silikone, Polyurethane, Acrylate bzw. Metharcylate, Polyethylen und Ethylencopolymere (wie z.B. Polyetylenvinylacetat), Polysulfone, Polyphenylsulfone oder Polyethersulfone, Polyetheretherketone, Polyphenyle (wie z.B. self-reinfored Polyphenylen (z.B. Proniva™).

Eine bevorzugte Herstellung eines Implantats 100 kann mit den folgenden Schritten ausgeführt werden:

PLLA (PLLA = polylactic acid, beispielsweise L214 S) wird in Chloroform gelöst (1 g/I). Der Wirkstoff 50 wird mit einem Gewichtsanteil zwischen 10 Gew.% und 60 Gew.% - vorzugsweise 30 Gew.% bis 40 Gew.% - bezogen auf das Polymer zugesetzt. Die Lösung wird mit aus dem Stand der Technik bekannten DES-Beschichtungsanlagen aufgesprüht. Die Schichtmasse beträgt nach dem Tempern (Lagerung bei erhöhter Temperatur und Vakuum zur Entfernung des Lösungsmittels) 100-10000 µg, vorzugsweise 100-2000 µg, vorzugsweise 600-900 µg. Die Auswahl der Polymermatrix 30 sowie die konkrete Beladung mit Wirkstoff 50 richten sich nach der für die eluierende Substanz angestrebte Freisetzungskinetik.

Der wirkstoffbeladene Trägerkörper 10 wird zur Abscheidung mit der undurchlässigen Beschichtung 15 auf einen Dorn (z.B. PTFE oder Silkonkautschuk) aufgesteckt und mit Parylen oder PES (siehe oben) erneut beschichtet.

So kann beispielsweise 1,5 g/l PES in Chloroform gelöst werden, um mit der Lösung anschließend eine Tauch- oder Spraybeschichtung durchzuführen. Parylene werden über eine Gasabscheidung bei Raumtemperatur bei vermindertem Druck aufgebracht bzw. appliziert. Die undurchlässige Beschichtung 15 kann günstigerweise eine Schichtdicken von 1 µm bis 5 µm, vorzugsweise 4 µm bis 5 µm aufweisen.

Durch die örtlich begrenzte Freisetzung des Wirkstoffs 50 können vorteilhaft Nebenwirkungen bei gleichzeitig hoher lokal verfügbarer Dosis minimiert werden.

Das wirkstofftragende Implantat 100 dient vorteihafterweise zur HCC-Behandlung mit vorzugsweise ausschließlich blutseitiger Wirkstofffreisetzung. Das Implantat 100 gibt den Wirkstoff 50 an das Blut ab, so dass es nicht nötig ist, das Implantat 100 in unmittelbarer Nähe eines Tumors zu platzieren. Das Implantat 100 kann deshalb z.B. einige Zentimeter vor dem Tumor fixiert werden. Der Wirkstoff 50 gelangt über den Blutfluss zum Zielort. Die äußere undurchlässigen Beschichtung 15 stellt sicher, dass der Wirkstoff 50, d.h. das Medikament, nicht an das in der Regel gesunde Gewebe, insbesondere die Gefäßwand, abgegeben wird und hier Nekrosen und Entzündungen hervorgerufen werden.

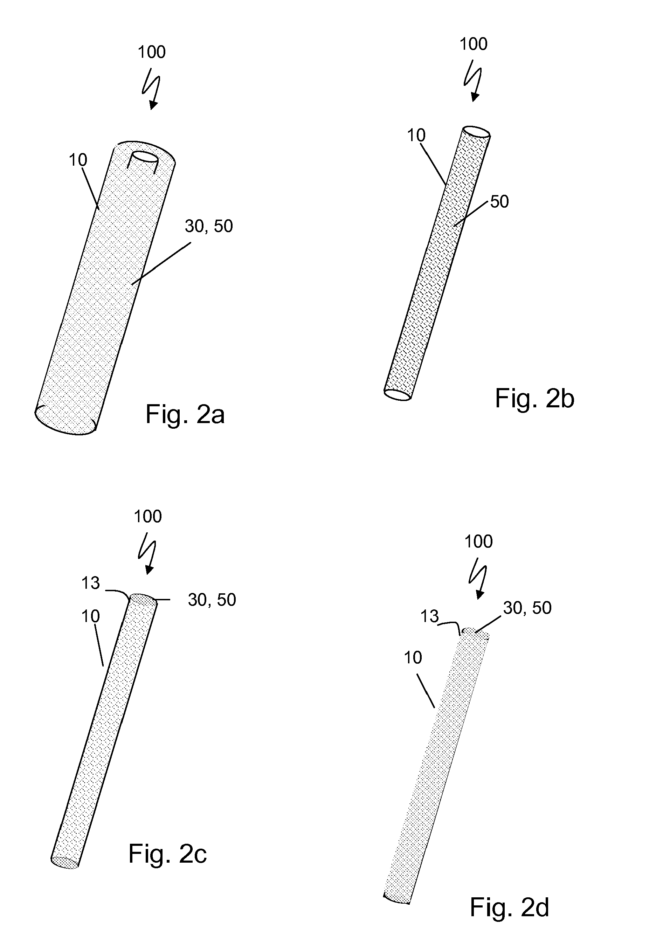

Zur Erläuterung einer weiteren günstigen Ausführungsform der Erfindung zeigen die Fig. 2a bis 2d verschiedene bevorzugte Ausgestaltungen eines wirkstoffbeladenen Implantats 100 mit einem Trägerkörper 10 und einem Wirkstoff 50 zur Freisetzung in einem Freisetzungsgebiet, vorzugsweise im Körpergewebe, in der Form eines Wirkstoffstifts. Der Wirkstoff 50 kann insbesondere zur Krebstherapie und/oder palliativen Behandlung vorgesehen und lokal und/oder regional gesteuert mit vorgebbarer Rate und/oder über eine vorgebbare Zeitdauer freisetzbar sein, wenn der Trägerkörper 10 in das Körpergewebe eingesetzt ist. Der Trägerkörper 10 kann mit einem Polymer beschichtet oder bedeckt sein, oder aus einem Polymer bestehen, das biologisch degradierbar ist und sich im Körper, in den das Implantat 100 appliziert wurde, auflöst. Gegebenenfalls kann das Implantat 100 auch ein nicht degradierbares Material umfassen oder aus einem solchen gebildet sein.

Eine Applikation des Implantats 100 kann durch einen geeignet modifizierten Katheder oder durch einen Trokar o.ä. vorgenommen werden. Beispielhaft ist eine günstige Vorrichtung in Fig. 2 dargestellt, mit der eine Mehrzahl von Implantaten 100 appliziert werden können.

Der Trägerkörper 10 des Implantats 100 kann hohl sein, wie in den Fig. 1a und 1b ersichtlich, oder massiv sein, wie den Fig. 1c und 1d zu entnehmen ist, und kann vorzugsweise im Gewebe positioniert werden. Der Wirkstoff 50 kann lokal in das Gewebe abgegeben werden.

Das wirkstofftragende Implantat 100 ist beispielsweise mit Zytostatika beladen, die bei systemischer Gabe durch ihre Art bzw. ihre Wirksamkeit zu schweren Nebenwirkungen führen würden. Die lokale Gabe macht es möglich, Dosierungen an einen Wirkungsort zu bringen, der systemisch nicht mit vergleichbarer Potenz erreichbar ist. Zusätzlich kann die Wirkstoffabgabe gesteuert ablaufen, so dass der Wirkstoff 50 über eine ausreichend lange Zeit zur Verfügung gestellt wird.

Vorzugsweise kann sich eine Elution des Wirkstoffs 50 an den folgenden Eckwerten orientieren, z.B. eine möglichst homogene Freisetzung des Wirkstoffs über mindestens 2 Wochen; ein Erreichen eines Dosis-Peak nach spätestens 24 Stunden; ein möglichst steiler Dosisabfall nach Ende des Behandlungszeitraums.

Die Erfindung erlaubt es im Bereich der lokalen Therapie, ein oder mehrere Medikamente gezielt in hoher Dosierung lokal zu verabreichen, ohne dabei durch Wirkungsweise und/oder Dosierungshöhe des Medikaments bedingte negative Nebenwirkungen in umliegendem Gewebe oder Körperregionen zu induzieren.

Die in den Fig. 2a bis 2d beschriebenen Varianten des Implantats 100 beschreiben einen Wirkstoffträger, im Folgenden kurz "Wirkstoffstift" genannt, der es vorteilhaft ermöglicht, sowohl einen Wirkstoff 50 in sehr hoher Dosierung lokal oder auch für regionale Therapieformen zu verabreichen, als auch einen erneuten Eingriff zum Entfernen des Implantats 100 aufgrund seines resorbierbaren Materialcharakters unnötig zu machen.

Ein weiterer Vorteil des bevorzugten, als Wirkstoffstift ausgebildeten Implantats 100 besteht in seiner Bauart, respektive seinen vorteilhaften mechanischen Eigenschaften, die es erlauben, auch bei hohem Wirkstoffgehalt das erfindungsgemäße Implantat 100 z.B. ins Gewebe zu implantieren. Im Stand der Technik erniedrigt ein hoher Wirkstoffgehalt in der Regel die mechanischen Eigenschaften eines Trägerkörpers stark.

Vorteilhaft kann mit dem bevorzugten Implantat 100 aufgrund resorbierbarer Materialien auf eine Reintervention zum Entfernen des Implantats verzichtet werden. Eine hohe, regionale und lokale Pharmakongabe ist möglich, ohne Nebenwirkungen auf z.B. gesundes, umliegendes Gewebe und/oder Organe zu induzieren.

Die mehreren beispielhaften Varianten zur Realisierung eines hoch wirkstoffbeladenen, als Wirkstoffstift ausgebildeten Implantats 100 mit ausreichenden mechanischen Eigenschaften, um z.B. keinen Schaden bei der Implantation oder während seiner Lebensdauer auftreten zu lassen, werden im Folgenden beschrieben. Eingangs der Beschreibung wurde auf die mögliche bevorzugte Materialauswahl seitens des Trägerkörpers 10, der Polymermatrizes 30 und des Wirkstoffs 50 oder der Wirkstoffe 50 eingegangen.

Fig. 2a zeigt eine erste bevorzugte Variante des bevorzugten als Wirkstoffstift ausgebildeten Implantats 100. Bei dieser Ausführungsform kann der Trägerkörper 10 des als Wirkstoffstift ausgebildeten Implantats 100 aus einem Vollmaterialstäbchen oder aus einem Rohr bestehen. Dieser Trägerkörper 10 liegt ummantelt mit einer wirkstoffinkorporierten Polymerschicht 30, in der der Wirkstoff 50 eingebettet ist, vor. Der Trägerkörper 10 kann bevorzugt aus bioresorbierbaren Polymeren und/oder aus bioresorbierbaren Metallen hergestellt werden.

Fig. 2b beschreibt eine zweite bevorzugte Variante eines bevorzugten als Wirkstoffstift ausgebildeten Implantats 100. Wie bei der ersten Variante kann der Trägerkörper 10 des als Wirkstoffstift ausgebildeten Implantats 100 auch aus einem Vollmaterialstäbchen oder aus einem Rohr bestehen. Die zweite Variante beschreibt einen vorzugsweise polymerfreien Ansatz, bei dem das Stäbchen / Rohr entweder eine aufgeraute oder eine mikrostrukturierte Oberfläche mit Kavitäten (Hohlräumen) besitzt, um Wirkstoff 50 aufnehmen zu können, anstatt den Wirkstoff 50 im Polymer eingelagert zu tragen, wie in Fig. 2a beschrieben wurde.

Fig. 2c beschreibt eine dritte bevorzugte Variante eines als Wirkstoffstift ausgebildeten Implantats 100. Die dritte Variante umfasst einen Trägerkörper 10, der aus einem Rohr mit einem inneren Hohlraum 13 gebildet ist. Optional kann die Mantelfläche dieses Rohres porös bzw. permeabel vorliegen. In den Hohlraum 13 des als Rohr ausgebildeten Trägerkörpers 10 wird eine wirkstoffbeladene Polymermatrix 30 als Kern eingebracht. Im Fall einer durchlässigen Mantelfläche kann der Wirkstoff 50 aus dem Kern im Hohlraum 13 des als Rohr ausgebildeten Trägerkörpers 10 nach außen ins Gewebe gelangen. Im Falle eines nicht-permeablen, als Rohr ausgebildeten Trägerkörpers 10 erfolgt die Abgabe des Wirkstoffs 50 gezielt aus den Enden des Trägerkörpers 10 des als Wirkstoffstift ausgebildeten Implantats 100. Hierbei kann optional auch ein Ende des als Rohr ausgebildeten Trägerkörpers 10 verschlossen werden, um eine weitere Steigerung der gezielten Wirkstoffabgabe zu ermöglichen.

Fig. 2d beschreibt eine vierte bevorzugte Variante eines bevorzugten als Wirkstoffstift ausgebildeten Implantats 100. Bei dieser Variante besteht der Trägerkörper 10 des als Wirkstoffstift ausgebildeten Implantats 100 aus einem Drahtgeflecht, Drahtgitter, oder einem Stent, in dessen inneren Hohlraum 13 ein Kern z.B. aus einem Polymer mit Wirkstoff 50 angeordnet ist. Vorteil dieser metallischen Komponente des Trägerkörpers 10 ist die Gewährleistung der mechanischen Eigenschaften, wie auch in vorstehend beschriebenen Varianten. In diesem speziellen Fall stellt sie zudem einen Schutz des Organismus vor bei der Degradation bzw. Fragmentierung des wirkstoffbeladenen Polymerkerns entstehenden Fragmenten dar. Der Wirkstoff 50 ist bevorzugt, wie in der dritten Variante, in eine wirkstoffbeladene Polymermatrix 30 im Hohlraum 13 eingebracht.

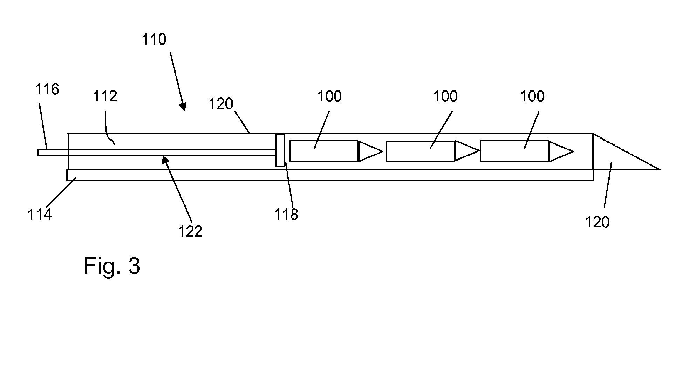

Fig. 3 illustriert ein vorteilhaftes Einführsystem 110 für einen oder mehrere als Wirkstoffstift ausgebildeten Implantate 100. Beispielhaft sind mehrere als Wirkstoffstift ausgebildete Implantate 100 hintereinander angeordnet in einem Innenraum 112 des Einführsystems 110 eingebracht. Proximal des proximalsten als Wirkstoffstift ausgebildeten Implantats 100 befindet sich ein Stempel 118, der außerhalb des Patienten mittels eines Manipulators 116 durch Verschieben gegen den Außenschaft 120 des Einführsystems 110 bewegt werden kann, um die hintereinander liegenden, als Wirkstoffstift ausgebildeten Implantate 100 distal aus dem Einführsystems 110 zu schieben. Eine distale Spitze 120 des beispielsweise als Katheter ausgebildeten Einführsystems 110 kann als Nadel ausgeführt werden, um einen Zugang in das Zielgewebe zu vereinfachen. Ein an sich bei Kathedern generell bekannter Führungsdraht zur besseren Applikation kann in einer Führung 114 vorgesehen sein und die distale Spitze 120 beim Einführen des Einführsystems 110 in den Körper überragen.

Der Vorschubmechanismus 122 verfügt zweckmäßigerweise über eine Rasterfunktion, die die Freisetzung jeweils eines einzelnen als Wirkstoffstift ausgebildeten Implantats 100 ermöglicht. Dadurch können in mehrere benachbarte Zielregionen jeweils ein oder mehrere als Wirkstoffstift ausgebildete Implantate 100 abgegeben werden. Das Einführsystem 110 bietet so beim Positionieren auch einen Schutz für die als Wirkstoffstift ausgebildeten Implantate 100 gegen mechanischen Abrieb oder Wirkstoffverlust an das umströmende Blut.

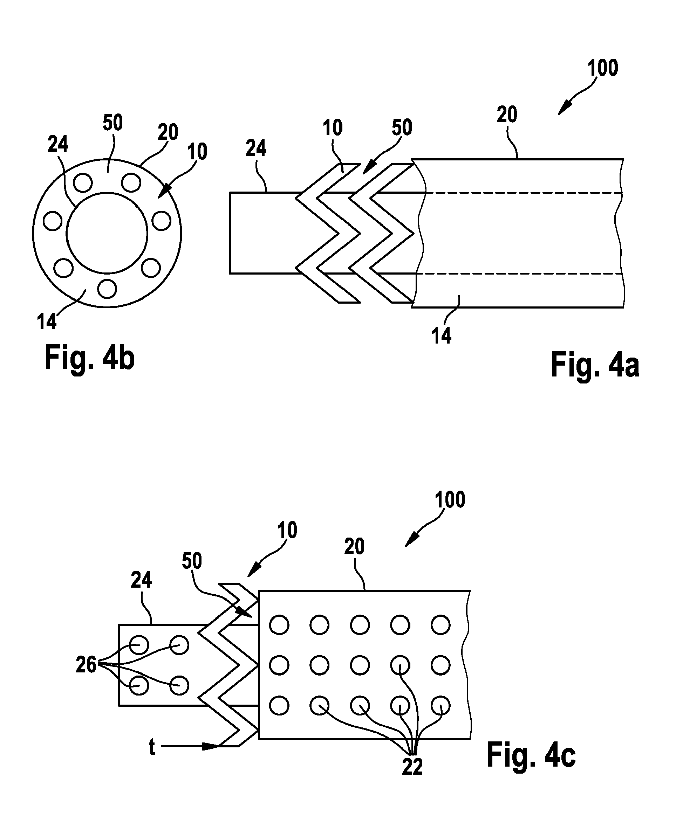

Die bestehenden Konzepte zum regionalen Wirkstoffeinsatz durch arteriell eingebrachte Wirkstoffdepots sind häufig nicht in der Lage, ausreichend große Mengen Wirkstoff in definierten Regionen einzubringen. Ein weiteres bevorzugtes Ausführungsbeispiel eines bevorzugten wirkstoffbeladenen Implantats 100 ist in zwei Varianten in Fig. 4a bis 4c dargestellt, das insbesondere zur RDD Therapie geeignet ist (RDD = regional drug delivery, lokale Wirkstofffreisetzung). Bevorzugt ist ein mehrlagiges Implantat 100 mit einem integriertem Stent als Trägerkörper 10.

Vorteilhaft ist das bevorzugte Implantat 100 in der Lage, große Mengen (z.B. 5 mg bis 100 mg, vorzugsweise 10 mg bis 100 mg) eines Wirkstoffes 50 in ein arterielles Gefäß abzugeben. Das Implantat 100 kann durch einen Katheter z.B. in ein arterielles Gefäß eingesetzt werden.

Das Implantat 100 besteht aus einem bedeckten Stent (Stent-Graft) als Trägerkörper 10, der vorzugsweise selbstexpandierbar ist. Dieser Trägerkörper 10 ist sowohl außen als auch innen z.B. durch einen polymeren Werkstoff 30 oder polymere Werkstoffsysteme, bedeckt, wie sie oben einleitend beispielhaft beschrieben sind. Zwischen der äußeren Abdeckung 20 und der inneren Abdeckung 24 des Trägerkörpers 10 befindet sich der Wirkstoff 50, wie aus Fig. 4a in teilweise aufgeschnittener Ansicht und in Fig. 4b im Querschnitt ersichtlich ist in einem Hohlraum 14 zwischen den Abdeckungen 20, 24. Die Funktion des Trägerkörpers 10, der vorzugsweise aus Nitinol (NiTi) bestehen kann, ist die Fixierung des Implantats 100 an der Gefäßwand. Der Wirkstoff 50 kann durch die äußere Abdeckung 20, durch die innere Abdeckung 24 oder gleichzeitig durch beide Abdeckungen 20 und 24 freigesetzt werden. Bevorzugt sind Ausführungsformen, bei denen der Wirkstoff 50 nicht an die Gefäßwand, sondern ausschließlich in das Lumen des Blutgefäßes abgegeben wird, in dem das Implantat 100 eingesetzt ist, um in der Gefäßwand unerwünschte Nebenwirkungen wie Entzündungen oder Nekrosen zu vermeiden. Hierbei kann die äußere Abdeckung 20 nichtpermeabel und die innere Abdeckung 24 permeabel ausgestaltet sein, um eine Diffusion des Wirkstoffs 50 durch die äußere nichtpermeable Abdeckung 20 zu unterbinden, bzw. durch die permeable innere Abdeckung 24 zu ermöglichen.

Grundsätzlich sind mehrere Varianten denkbar. Bei einer ersten Variante können die Abdeckungen 20 und/oder 24 zumindest teilweise aus abbaubaren Materialen, z.B. aus degradierbarem Polymer, Fibrin, Acryl oder Gewebe, wobei der Wirkstoff 50 durch Degradation der Abdeckungen 20 und/oder 24 freigesetzt wird. In einer zweiten Variante kann eine Freisetzung des Wirkstoffs 50 durch perforierte Grafts, d.h. Perforierungen der Abdeckungen 20 und/oder 24 erfolgen. In einer dritten Variante kann der Trägerkörper 10 Aus abbaubaren Werkstoffen (degradable Polymere oder Metalle) gebildet sein.

Das Implantat 100 gemäß der ersten Variante stellt einen selbstexpandierenden Nitinol-Graft-Stent dar. Die Räume zwischen der äußeren, adluminalen Abdeckung 20 des Trägerkörpers 10 sind mit Wirkstoff 50 ausgefüllt. Die Beschichtungsmaterialien bestehen wie oben beispielhaft aufgelistet aus z.B. biologisch abbaubaren Werkstoffen. Im Rahmen des Abbauprozesses der Abdeckung 20 wird der Wirkstoff 50 freigesetzt. In einer bevorzugten Ausführungsform wird der Wirkstoff 50 ausschließlich luminal abgegeben. Hierbei ist die innere, (ad)luminale Abdeckung 24 derart degradierbar, dass der Wirkstoff 50 während des Degradationsprozesses freigesetzt wird, während die äußere (nicht-permeable) Abdeckung 20 entweder nicht degradierbar ist oder so langsam degradiert, dass der Degradationsprozess erst dann signifikant einsetzt, nachdem der Wirkstoff 50 bereits vollständig in das Lumen abgegeben wurde.

Das Implantat 100 stellt in der zweiten Variante wie bei der ersten Variante einen Graft-Stent dar; jedoch wird der Wirkstoff 50 durch die strukturelle Perforierung 22 bzw. 26 der Abdeckungen 20 und/oder 24 freigesetzt (z.B. in Richtung Gefäß oder in die Blutbahn oder in beide Richtungen). Dies ist in Fig. 4c dargestellt. Die Räume zwischen der äußeren und inneren Abdeckung 22 und 24 des Trägerkörpers 10 sind mit Wirkstoff 50 ausgefüllt wie bei der ersten Variante. Bei den Abdeckungen 20, 24 kann es sich um permanente oder degradable Materialien handeln; bei letzteren wird der Wirkstoff 50 (im Gegensatz zur ersten Variante) nicht durch Degradation, sondern überwiegend durch Diffusion freigesetzt. Wiederum kann die murale (äußere) Abdeckung 24 vorzugsweise nichtpermeabel gestaltet sein, um die Gefäßwand nicht unnötig zu belasten.

Eine dritte und vierte Variante (nicht abgebildet) kann gleich wie die erste und zweite Variante ausgebildet sein, jedoch besteht der Trägerkörper 10 aus degradierbaren Werkstoffen wie z.B. abbaubaren Polymere oder Metallen. In diesem Fall bestehen die Abdeckungen 20, 24 vorzugsweise aus degradierbaren Materialien, wodurch ein volldegradierbares Hybrid entsteht.

Dieses Konzept erlaubt die Implantation von großen Wirkstoffmengen in bestimmten Regionen der Arterien. Die Realisierung einer Implantation ist durch ein geeignet modifiziertes Einführsystem (Stent Delivery System, SDS) praktizierbar.

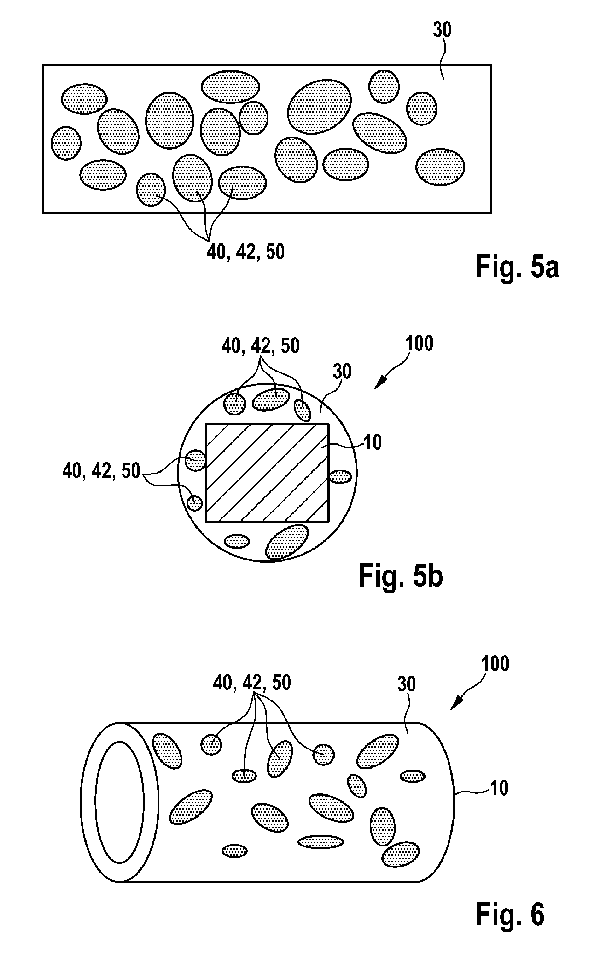

Ein weiteres Ausführungsbeispiel eines bevorzugten Implantats 100 ist in den Fig. 5a, 5b und Fig. 6 in zwei Varianten erläutert, das besonders bevorzugt zur Durchführung einer TACE-Behandlung mit einem RDD-Implantat 100 geeignet ist (TA-CE = TransArterielleChemoEmbolisation), mit zusätzlichem Potential zur lokalen oder regionalen Freisetzung eines Wirkstoffs 50.

Mit der TACE-Therapie wird in der konventionellen Onkologie ein palliatives Vorgehen darstellt, welche in der endokrinologisch-onkologischen Therapie von neuroendokrinen Tumoren grundsätzlich einen potentiell kurativen Ansatz hat. Sie ist nicht nur auf die Beseitigung anderweitig nicht beherrschbarer Symptome beschränkt (z.B. Hypoglykämien). Lebermetastasen eines Tumors werden durch arterielle Gefäße der Leber versorgt. Das hepatische arterielle Gefäßsystem trägt zu etwa 30% der Sauerstoffversorgung bei, 70% stammen aus der portalvenösen Blutversorgung über die Pfortader (V. portae). Deshalb toleriert die Leber einen gezielten "Infarkt" der arteriellen Blutzufuhr durch Embolisation einzelner Segmente (partiell selektiv), eines ganzen Leberlappens oder sogar der gesamten Leber. Daher wird die Leberembolisation bei bösartigen Grunderkrankungen in der Onkologie mit unterschiedlichem Erfolg seit Jahren angewandt, z.B. bei HCC (HCC = hepatocelluläres Leberzell-Carcinom). Hier handelt es sich jedoch zumeist um infiltrativ destruierend wachsende Tumoren, die rasch die normale Leberfunktion beeinträchtigen. Die Metastasen neuroendokriner Tumoren, die zumeist langsam verdrängend ("pusher") wachsen und die Leberfunktion kaum beeinträchtigen, eignen sich fast immer zur Behandlung durch Embolisations-Therapien.

Allerdings hält in den bekannten Embolisationstherapien aufgrund der unterschiedlichen Materialien für die Embolisation, vor allem aber der von Tumor zu Tumor unterschiedlichen Proliferationstendenz der Tumor-Gefäße der Erfolg einer Embolisation z.T. nur wenige Wochen an. Wiederholungen sind kurzfristig möglich, häufig nötig und auch präventiv bei Minimalbefunden indiziert. Neben der Gefahr einer zeitlichen Befristung des Therapieerfolges der Embolisation ist auch in der Chemotherapie, welche in einem weiteren Interventionsschritt und in einer hochdosierten Einmalgabe durchzuführen ist, eine weitere Belastung für den Patienten und das Gesundheitswesen.

Das erfindungsgemäße bevorzugte Implantat 100 mit einer Polymermatrix 30 vereint vorteilhaft den Ansatz der TACE-Therapie, speziell die Embolisation, mit einer Chemo- respektive Medikamententherapie innerhalb des Implantats 100. Hierbei sind sowohl mehrere Medikamente als Wirkstoff 50 zeitlich getrennt und in variabler Dosierung regional und lokal verabreichbar, sowie Größe und Freisetzungsrate der die Embolisation verursachenden Embolisationspartikel 40 steuerbar. Bei der TACE-Matrix können die Embolisationspartikel 40 als Trägerkörper für das Medikament dienen. Auf einem Trägerkörper 10 werden die Embolisationspartikel 40 in das Gefäß eingebracht, und innerhalb der Embolisationspartikel 40 kann quasi als AddOn Wirkstoff 50 eingebettet sein.

Durch das Zusammenspiel aus Medikamenten- und Partikelfreisetzung innerhalb nur eines Eingriffes ist ein größerer und länger anhaltender Therapieerfolg gegeben. Somit kann eine Senkung der Morbidität und eine Verringerung der Therapiekosten erzielt werden.

Bevorzugt ist eine erfindungsgemäße Materialmatrix, vorzugsweise eine Polymermatrix 30, die entweder auf einem Trägerkörper 10, etwa einem Stent, wie in Fig. 5b dargestellt ist, aufgebracht oder ohne Verwendung eines separaten Trägers z.B. als Schlauch (Fig. 6) in ein Blutgefäß eingebracht werden kann, so dass die Polymermatrix 30 selbst den Trägerkörper 10 bildet. Das bevorzugte Implantat 100 vereint den Ansatz der TACE-Therapie (Embolisation) mit einer Chemo- respektive Medikamententherapie vorteilhaft innerhalb eines Bauteils.

So wird bei vorgestelltem Implantat 100 der Wirkstoff 50 nicht einmalig intraarteriell appliziert, sondern der Wirkstoff 50 liegt inkorporiert in einer oder in mehreren der in der TA-CE-Matrix (Polymermatrix 30) verwendeten Polymeren vor. Dies hat zudem den Vorteil, mehrere Medikamente als Wirkstoff 50 zeitlich unabhängig, örtlich gezielt und genau dosiert verabreichen zu können, ohne dabei Nebenwirkungen zu induzieren.

Zudem kann mit dem bevorzugten Implantat 100 erreicht werden, nicht eine einmalige Embolisation - bei der ein anhaltender Therapieerfolg unklar ist - zu verursachen, sondern über eine kontrollierte Freisetzung, bevorzugt zeitlich und/oder mengenmäßig, von embolisationverursachenden Embolisationspartikel 40 eine "sequentielle Embolisation" herbeizuführen. Somit können wiederholte Eingriffe reduziert oder günstigstenfalls sogar. vollständig vermieden werden.

Vorteilhaft findet bei dem Implantat 100 keine Trennung zwischen Chemo- und Embolisationstherapie statt. Eine exakte Dosierung und zeitliche Freisetzung der embolisationverursachenden Embolisationspartikel 40 im Gegensatz zur Einmalgabe nach konventioneller Therapie kann erreicht werden. Es kann eine zur Embolisation begleitende Pharmakon- bzw. Chemotherapie, realisiert werden, bei der regional und lokal gezielte Medikamentengaben über einen längeren Therapiezeitraum freigesetzt werden können und somit nicht zu Nebenwirkungen, wie bei der herkömmlichen Therapieform mit hochdosierten Einzelgaben, führen. Über eine geeignete Materialauswahl ist das Implantat 100 in einem großen Zeitfenster vollständig resorbierbar herstellbar, eine Intervention zum Entfernen des Implantats 100 entfällt somit.

Eine Komponente des Implantats 100 ist die Polymermatrix 42 (s. schematische Darstellung in Fig. 5a). Diese stellt die zur Embolisation freizusetzenden Embolisationspartikel 40 in den bevorzugten Größen zwischen 2 µm und 200 µm dar. Eingearbeitet sind diese Embolisationspartikel 40 in die Polymermatrix 30. Die Embolisationspartikel 40 können z.B. über Sprühtrocknungsprozesse hergestellt werden. Die Embolisationspartikel 40 können aus degradierbaren oder permanenten Polymeren gebildet sein oder auch aus einem superabsorbierenden Material bestehen.

Eine Oberflächenmodifizierung der Embolisationspartikel 40 mit dem Ziel, eine chemische Bindung mit dem Material der Polymermatrix 30 zu unterbinden, ist optional und kann entsprechend der verwendeten Polymerpaarungen für die Matrix 42, die Embolisationspartikel 40 und die Polymermatrix 30 evaluiert werden. Die Matrix 42 kann auch entfallen und die Embolisationspartikel 40 direkt in der Polymermatrix 30 eingebettet sein.

Je nach Füllgrad der Polymermatrix 30 mit der ersten Matrix 42 kann die Anzahl der embolisationsverursachenden Embolisationspartikel 40 gesteuert werden. Über die Resorptions-, Degradationsgeschwindigkeit der Polymermatrix 30 kann die Dosierung und damit auch der Therapiezeitraum der Embolisation eingestellt werden.

Zusätzlich zu der reinen Partikelfreisetzung können zur Unterstützung der Therapie beide Matrices 30, 42 mit Wirkstoff 50 beladen sein. Gerade durch mit Wirkstoff 50 beladene Embolisationspartikel 40 kann der Wirkstoff 50 sehr lokal appliziert werden.

Eine günstige Variante in Fig. 5b zeigt eine TACE-Matrix, bestehend aus Polymermatrix 30 und Embolisationspartikel 40 bzw. Matrix 42, auf einem Stent als Trägerkörper 10. Idealerweise liegt bei dieser Variante nur die innere luminale Stentseite beschichtet vor, welches über prozesstechnische Modifikationen bei der Herstellung einfach realisierbar ist.

Zudem ist es möglich, die TACE-Matrix zwischen zwei Stents als Trägerkörpern 10 zu positionieren (nicht dargestellt)

In einer zweiten Variante ist die TACE-Matrix als selbstragender Schlauch in eine Gefäßwand geklebt oder gepresst, so dass die Polymermatrix 30 selbst den Trägerkörper 10 bildet. Eine Applikation kann mittels einem Ballonkatheter erfolgen mit einem Haftvermittler zur Adhäsion mit der Gefäßwand, wobei z.B. Fibrin, Acrylate denkbar sind. Das Aufbringen einer "Schützhülle" über einen Transport-Stent, vergleichbar zur Hülle bei selbstexpandierbaren Stents, ist sinnvoll, um ein Positionieren und/oder Fixieren und/oder Verkleben des Schlauches an der gewünschten Stelle im Freisetzungsgebiet realisieren zu können. Optional kann dieser Schlauch selbstverständlich auch auf einem Stent als Trägerkörper 10 angebracht sein.

Eine Auflistung geeigneter Polymere zur Anwendung als TACE-RDD-Matrix ist, wie bereits auch bei den anderen Ausführungsbeispielen erwähnt, eingangs der Beschreibung aufgeführt, speziell unter dem Aspekt unterschiedlicher Resorptions-, bzw. Degradationsgeschwindigkeit.

Während die unter dem Punkt "schnell bioresorbierbare / degradierbare Materialien" und unter dem Punkt "langsam resorbierbare / bioresorbierbare / degradierbare Polymere" aufgeführte Materialauswahl darauf abzielen, neben der regionalen Pharmakontherapie einen temporären Verschluss versorgender Gefäße zu erzeugen (temporäre Embolisation), können auch geeignete permanente Polymere (s. Punkt "permanente Polymere") gewählt werden oder als Besonderheit die eingebetteten Embolisationspartikel 40 auch aus sog. superabsorbierbaren Materialien bestehen, die nach ihrer Freisetzung in wässrigen Systemen quellen.

Günstige Superabsorber-Materialien sind z.B. typische Materialien wie Polyethylenoxid, Polyvinylalkohol, Polyacrylsäure (vernetzt, teilvernetzt; teilneutralisiert), Polyacrylat, Polymerblends aus Polyacrylsäure und Natriumacrylat, Nicht-ionische Polymere (z.B. vernetztes Polyacrylamid), Polycarboxylate, Polycyanacrylat, Polyvinylbutyral, etc. Generell versteht sich unter der Klasse der Superabsorber vernetzte oder teilvernetzte sowie Oberflächenvernetzte oder "Bulk" und "Core" vernetzte Polymerer oder Polymerblends. Typische Vernetzungsmittel sind hierbei z.B. Tetraallylethoxyethan oder 1,1,1-Trimethylolpropantricrylat (TMPTA).

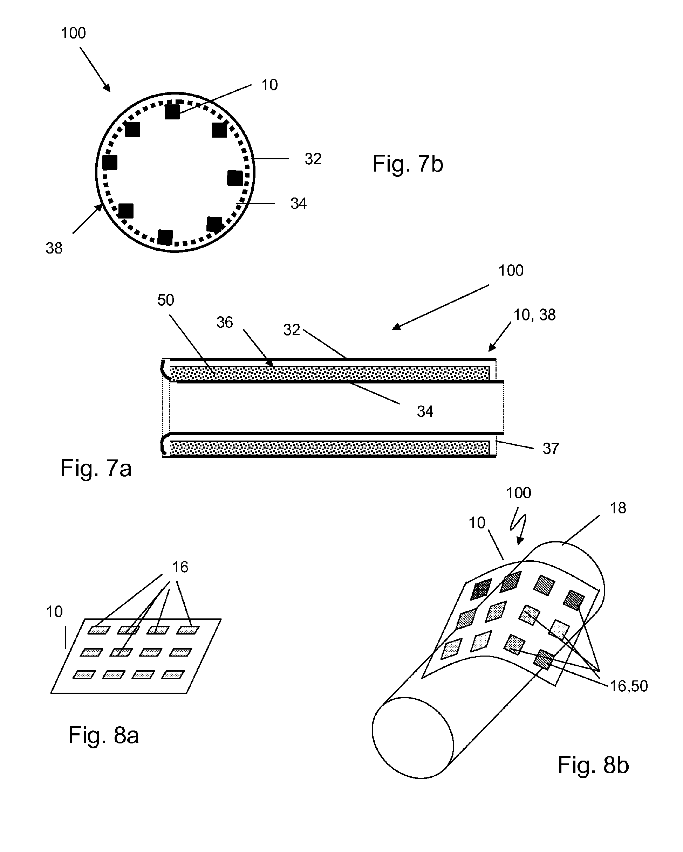

Eine weitere vorteilhafte Weiterbildung der Erfindung ist in den Fig. 7a und 7b dargestellt. Das bevorzugte Implantat 100 umfasst in dieser Ausführung ein schlauchartiges Material, welches umgestülpt werden kann und in dem dabei gebildeten Hohlraum Wirkstoff 50 aufnehmen kann. Besonders bevorzugt kommt diese Weiterbildung bei der Behandlung von inoperablen HCC zum Einsatz.

Das wirkstofftragende Implantat 100 kann mit Zytostatika als Wirkstoff 50 beladen werden, die bei systemischer Gabe durch ihre Art bzw. ihre Wirksamkeit zu schweren Nebenwirkungen führen würden. Die lokale Gabe macht es möglich Dosierungen an einen Wirkungsort zu bringen, der systemisch nicht mit vergleichbarer Potenz erreichbar ist. Zusätzlich kann die Wirkstoffabgabe gesteuert ablaufen, so dass der Wirkstoff 50 über eine ausreichend lange Zeit zur Verfügung gestellt wird. Die Elution des Wirkstoffs 50 kann sich an den folgenden Eckwerten orientieren:
- möglichst homogene Freisetzung des Wirkstoffs 50 über mindestens zwei Wochen;
- Dosis-Peak sollte nach spätestens 24 Stunden erreicht sein;
- der Dosisabfall nach Ende des Behandlungszeitraums sollte möglichst steil sein.

Die Herstellung eines bevorzugten schlauchförmigen Implantats 100 kann mit folgenden Schritten erfolgen. Dabei kann das Implantat 100 selbsttragend sein oder einen Trägerkörper 10 als stützendes Gerüst, z.B. aus Nitinol oder anderen Metallen oder polymeren Werkstoffen, besitzen.

Der Schlauch 38 besteht aus einem flexiblen polymeren Material und wird z.B. zur Hälfte umgeschlagen (Fig. 7a), so dass sich ein äußerer Schlauchabschnitt 32 und ein innerer Schlauchabschnitt 34 bildet. Der Hohlraum 36 zwischen den Schlauchabschnitten 32, 34 wird als Wirkstoffreservoir für den Wirkstoff 50 benutzt. Bei diesem Ansatz ist es nötig, für ein z.B. 18 mm langes Schlauchimplantat 100 einen z.B. 36 mm langen Schlauch 38 herzustellen.

Alternativ kann der Schlauch 38 als koaxialer Doppelschlauch mit Innenschlauch (entsprechend Schlauchabschnitt 34) und Außenschlauch (entsprechend Schlauchabschnitt 32) extrudiert werden. Über einen Ringspalt kann zusätzlich ein Coextrudat auf dem Innenschlauch aufgebracht werden. Dabei kann in die Polymerschmelze des Innenschlauches der Wirkstoff 50 eingebracht werden. Der Innenschlauch bildet dann eine wirkstoffbeladene Polymermatrix 30. Fig. 7b zeigt einen Querschnitt eines derartigen coextrudierten Schlauchs 38. Als Trägerkörper 10 kann z.B. ein Stent im Inneren des Schlauchs 38 vorgesehen sein.

Das Implantat 100 gibt über den Schlauch 38, durch den das Blut fließt, nur Wirkstoff 50 an das Blut und nicht an die Gefäßwand oder das Gewebe am Implantationsort ab. Als Schlauchmaterial ist eine Reihe von vorzugweise thermoplastischen Werkstoffen denkbar.

Das Implantat 100 kann auch in Form einer Spirale gebildet sein (nicht dargestellt). Dabei besteht die Spirale aus einem Rohr, das mit Wirkstoff oder einer Wirkstoffformulierung befüllt ist. Als Material ist Nitinol besonders geeignet. Die Spirale verbleibt im Gefäß, durch die Öffnung wird der Wirkstoff abgegeben. Durch Kerben über die Länge der Spirale kann die Wirkstofffreisetzung beeinflusst werden.

Wirkstoffe mit zytostatischen Eigenschaften, die für das Implantat eingesetzt werden können, sind bevorzugt Doxorubicin, Epirubicin, Cisplatin, Mitomycin C (als Einzelsubstanz oder in Kombination), oder andere eingangs aufgelistete Wirkstoffe 50.

Der Wirkstoff 50 kann als Reinsubstanz oder als Formulierung eingesetzt werden. Dabei können die folgenden Polymere verwendet werden, um eine geeignete Freisetzungskinetik zu realisieren: Polylactide, wie poly-L-lactide, poly-D-Lactide, Polyglycolide, Polydioxanone, Polycaprolactone, Poly(hydroxybutyrate), und Polygluconate, Polylactic-acid-Polyethylene-Oxid-Copolymere, Saccharide wie modifizierte Cellulose, Alginat Chitosan, Polypetide wie Collagen oder Matrigel®, Polyanhydride, Polyorthoester, Poly(alpha-hydroxy Säure) und Kombinationen.

Ebenso können nichtabbaubare Polymere verwendet werden, wie etwa Silikone, Polyurethane, Acrylate bzw. Metharcylate, Polyethylen und Ethylencopolymere wie Polyetylenvinylacetat, Polysulfone, Polyphenylsulfone oder Polyethersulfone, Polyetheretherketone, Polyphenyle wie selbst-verstärktes (self-reinforced) Polyphenylen (z.B. Proniva^{™}.

Auch liposomale Verkapselung oder Mikroverkapselung sind für eine zielgerichtete Einstellung des Wirkstofffreisetzungsverhaltens geeignet. Ferner können Cyclodextrine, insbesondere beta-Cyclodextrin oder 6-O-Palmitoyl-L-ascorbinsäure als Additiv bei Verwendung von Polymeren aus der vorstehenden Auflistung eingesetzt werden.

Ein vorteilhaftes Herstellungsverfahren des Schlauchs 38 kann beispielhaft mit folgenden Schritten erfolgen; bei anderen Materialien oder Zusammensetzungen können auch andere Verfahrensparameter gelten:
Polyethylenglycol (Molgewicht 4000 g/mol) wird fein gemörsert und fünf Tage bei vermindertem Druck (Wasserstrahlvakuum, 17 Torr) im Exikator über Phosphorpentaoxid getrocknet. In 2-ml-Eppendorf-Reagiergefäßen werden je 1 g PEG (Polyethylenglykol) eingewogen und bei 62°C geschmolzen. Zu der Schmelze werden 100 - 200 µl HMDI (Hexamethyldiisocyanat) zugegeben. Die zweiphasige Mischung wird unter Verwendung eines Vortex gut durchmischt und für 1,5 Std. bei 62°C inkubiert. Die Topfzeit beträgt 30 Minuten. Die Schmelze wird mit einer Pasteurpipette gerührt. Über die Topfzeit wird das Material aus der Schmelze heraus verarbeitet.

Dazu werden Template (Glas oder Metallstäbe mit gewünschtem Innendurchmesser) mit der Schmelze beschichtet und bei Raumtemperatur für 12 Stunden gelagert. Nach Ablauf der Lagerung werden die Schläuche vom Templat gelöst. Das Ablösen wird erleichtert, wenn die Schläuche 38 in bidestilliertem Wasser quellen können. Es liegen Schläuche 38 mit homogener Wandstärke und Ausmaßen vor, die zugeschnitten und sterilisiert werden können. Eine Dampfsterilisation ist möglich.

Diese Schläuche 38 können zur Hälfte umgeschlagen werden (Fig. 7a). Der entstehende Hohlraum zwischen dem äußeren und dem inneren Schlauchabschnitt 32, 34 kann mit Wirkstoff 50 befüllt werden. Mit der Öffnung 37 voran werden die Implantate 100 ins Blutgefäß zum Freisetzungsgebiet gebracht.

Die Herstellung eines coextrudierten Schlauchs 38 (Fig. 7b) mit einem wirkstofftragenden Innenschlauch 34, über den ein undurchlässiger Außenschlauch 32 durch Coextrusion gebracht wird, kann mit folgenden Schritten erfolgen:

In den Schlauch, der den inneren Schlauchabschnitt 34 bilden soll, wird eine Wirkstofflösung mit Wirkstoff 50 gebracht. Nach dem Verdunsten des Lösungsmittels besitzt der Schlauch eine mit Wirkstoff 50 ausgekleidete Innenfläche. Zur Beeinflussung der Freisetzung kann die Lösung neben Wirkstoff 50 und Lösungsmittel auch ein Polymer oder Agens aus der oben in diesem Ausführungsbeispiel genannten Aufzählung oder der eingangs aufgeführten Auflistung besitzen, mit deren Hilfe eine Verzögerung der Freisetzung realisiert werden kann. Das zuletzt genannte Verfahren besticht durch seine einfachen Aufbau und kann deshalb bevorzugt werden.

Durch die örtlich begrenzte Freisetzung des Wirkstoffs 50 werden Nebenwirkungen bei gleichzeitig hoher lokal verfügbarer Dosis minimiert. Das Implantat 100 gibt den Wirkstoff 50 an das Blut ab, so dass es nicht nötig ist, das Implantat 100 in unmittelbarer Nähe eines Tumors zu platzieren. Das Implantat 100 kann deshalb einige Zentimeter vor dem Tumor fixiert werden. Der Wirkstoff 50 gelangt über den Blutfluss zum Zielort. Die äußere Ummantelung stellt sicher, dass das Medikament nicht an das, in der Regel nicht erkrankte, Gewebe - insbesondere die Gefäßwand - abgegeben wird und hier Nekrosen und Entzündungen hervorgerufen werden.

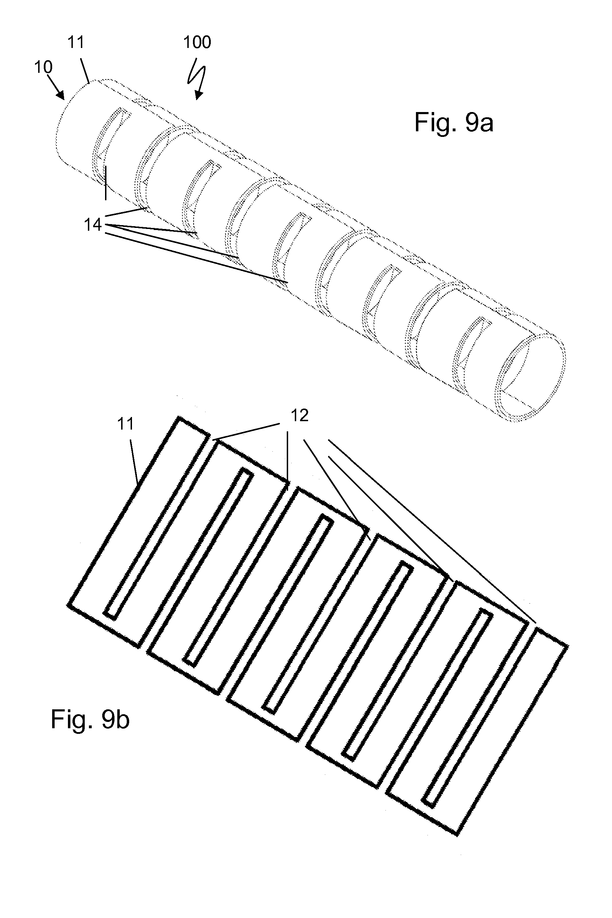

Die Fig. 8a, 8b und 9a, 9b zeigen Varianten einer weiteren bevorzugten Ausgestaltung eines erfindungsgemäßen wirkstoffbeladenen Implantats 100.

Fig. 8a und 8b zeigen eine Einbettung eines Wirkstoffs 50 in eine Folie 11 als Trägerkörper 10, die z.B. aus Nitinol oder aus anderen Materialien gebildet sein kann, wie z.B. Edelstahl 316 L, CoCr, Mg. Die Folie 11 weist bevorzugt eine Dicke zwischen 40 µm und 1000 µm auf, besonders bevorzugt um 200 µm, und wird über einen Tiefziehprozess mit Vertiefungen 16 versehen, in die der Wirkstoff 50 als Reinsubstanz oder als Formulierung mit den eingangs und/oder unten genannten Polymeren oder anderen Hilfsmitteln wie Polyvinylpyrrolidon, PEG (Polyethylenglykol), Cyclodextrinen, PVA (Polyvinylalkohol) in unterschiedlichen Verseifungsgraden, Hydrogelen wie Hyaluronsäure, Alginat, Chitosan, Gelatine,... 6-O-Palminyl-L-Ascorbinsäure oder Laurinsäure zusammen eingebracht wird.

Die Folie 11 wird, mit den Vertiefungen 16 nach innen ragend, um einen Ballon 18 gerollt (Fig. 8b) und mit einem Schutzschlauch fixiert. Nach dem Positionieren im Blutgefäß wird der Schutzschlauch zurückgezogen und die Folie 11 mit dem Wirkstoff 50 so freigesetzt.

Es wird ein Wirkstoff 50 mit zytostatischen Eigenschaften bevorzugt, wie z.B. Doxorubicin, Epirubicin, Cisplatin, Mitomycin C (als Einzelsubstanz oder in Kombination) oder eingangs genannte Materialien, die in degradierbare Polymere eingebracht werden wie z.B. Poly-L-Lactid, Poly-D-Lactid, Polyglycolid, Polydioxanone, Polycaprolacton, und Polygluconat, Polylactid-Säure - Polyethylene-Oxid-Copolymere, modifizierte Cellulose, Collagen, Poly(hydroxybutyrat), Polyanhydrid, Polyphosphoester, Poly(amino-Säuren), Poly(alpha-hydroxy-Säure) und Kombinationen.

Ebenso können nichtabbaubare Polymere vorgesehen sein wie z.B. Silikone, Polyurethane, Acrylate bzw. Metharcylate, Polyethylen und Ethylencopolymere wie Polyetylenvinylacetat, Polysulfone, Polyphenylsulfone oder Polyethersulfone, Polyetheretherketone, Polyphenyle wie selbst-verstärktes (self-reinforced) Polyphenylen (z.B. Proniva^{™}).

Beispielhaft wird eine etwa 200 µm dicke Folie 11 mit z.B. 18 mm Länge und z.B. 13 mm Breite in einer Stanzvorrichtung mit kleinen Wannen oder Vertiefungen 16 versehen. Günstige Abmessungen für die Folie 11 liegen z.B. im Bereich von 8 mm bis 30 mm Länge und im Bereich von 4 mm bis 30 mm Breite. Die Vertiefungen 16 weisen vorzugsweise Abmessungen von 1 mm bis 2 mm Länge und 1 mm bis 2 mm Breite oder, bei runder Ausgestaltung, einen entsprechenden Durchmesser auf. Die Foliendicke liegt vorzugsweise im Bereich zwischen 60 µm bis 3000 µm.

In die Vertiefungen 16 wird der Wirkstoff 50 eingebracht, z.B. durch Tauchen und Oberfläche abstreifen; Sprühbeschichten; Ink-Jet Verfahren, bei dem Löcher separat gefüllt werden; Einpressen von Wirkstoffbeats oder Presslingen.

Fig. 9a und 9b zeigen eine Variante, bei der die Folie 11 mit Schlitzen 12 versehen wird (Fig. 9a) und aufgerollt wird. In die durch die Schlitze 12 gebildeten Hohlräume 14 kann Wirkstoff 50 eingebracht werden, oder diejenige Seite, welche die Innenseite der aufgerollten Folie 11 bilden soll, wird mit Wirkstoff 50 in reiner Form oder in einer Polymermatrix (nicht dargestellt), beschichtet. Denkbar ist auch, einen Wirkstoffstift (s. Fig. 2a-2d) in das Innere der aufgerollten Folie 11 einzubringen. Die aufgerollte Folie 11 bildet einen Trägerkörper 10 für den Wirkstoff 50. Die Folie 11 kann auch aus einem degradierbaren Material gebildet sein.

Durch die örtlich begrenzte Freisetzung des Wirkstoffs 50 werden Nebenwirkungen bei gleichzeitig hoher lokal verfügbarer Dosis minimiert.

Das Implantat 100 gibt den Wirkstoff an das Blut ab, so dass es nicht nötig ist, das Implantat 100 in unmittelbarer Nähe des Tumors zu platzieren. Das Implantat 100 kann deshalb einige cm vor dem Tumor fixiert werden. Der Wirkstoff gelangt über den Blutfluss zum Zielort. Die Bauart des Implantats 100 stellt sicher, dass der Wirkstoff 50 nicht an das in der Regel gesunde Gewebe abgegeben wird und dort Nekrosen und Entzündungen hervorgerufen werden.

## Patentansprüche

1. Wirkstoffbeladenes Implantat (100) mit einem Trägerkörper (10) und wenigstens einem Wirkstoff (50) zur Freisetzung in einem Freisetzungsgebiet, **dadurch gekennzeichnet, dass** der Wirkstoff (50) zur Krebstherapie und/oder palliativen Behandlung vorgesehen ist und im bestimmungsgemäßen Wirkzustand des Implantats (100) in einem Körper eines Lebewesens lokal und/oder regional gesteuert mit vorgebbarer Rate und/oder über eine vorgebbare Zeitdauer freisetzbar ist.

2. Implantat nach Anspruch 1, **dadurch gekennzeichnet, dass** der Trägerkörper (10) biodegradierbar ausgebildet ist.

3. Implantat nach Anspruch 1 oder 2, **dadurch gekennzeichnet, dass** der Wirkstoff (50) in eine Polymermatrix (30) eingebettet ist, aus der der Wirkstoff (50) durch Degradation der Polymermatrix (30) freisetzbar ist.

4. Implantat nach Anspruch 3, **dadurch gekennzeichnet, dass** der Trägerkörper (10) wenigstens bereichsweise mit der Polymermatrix (30) beschichtet ist.

5. Implantat nach einem der vorhergehenden Ansprüche, **dadurch gekennzeichnet, dass** der Wirkstoff (50) in Vertiefungen (16) und/oder Hohlräumen (13, 14) des Trägerkörpers (10) angeordnet ist.

6. Implantat nach einem der vorhergehenden Ansprüche, **dadurch gekennzeichnet, dass** die Polymermatrix (30) wenigstens bereichsweise den Trägerkörper (10) bildet.

7. Implantat nach einem der vorhergehenden Ansprüche, **dadurch gekennzeichnet, dass** der Wirkstoff (50) zwischen zwei Polymerschichten (20, 24) angeordnet ist, welche den hohlen Trägerkörper (10) adlumal und ablumal umgeben.

8. Implantat nach einem der vorhergehenden Ansprüche, **dadurch gekennzeichnet, dass** der Trägerkörper (10) durch einen Schlauch (38) gebildet ist, der bereichsweise umgestülpt ist, wobei der Wirkstoff (50) im Bereich einer durch die Umstülpung gebildeten Hohlraum (36) angeordnet ist.

9. Implantat nach einem der vorhergehenden Ansprüche, **dadurch gekennzeichnet, dass** in einer Polymermatrix (30, 42) freizusetzende Embolisationspartikel (40) vorgesehen sind.

10. Implantat nach Anspruch 9, **dadurch gekennzeichnet, dass** die Polymermatrix (30) und/oder die Embolisationspartikel (40) mit Wirkstoff (50) beladen sind.

11. Implantat nach einem der vorhergehenden Ansprüche, **dadurch gekennzeichnet, dass** eine wirkstoffundurchlässige Deckschicht (15) vorgesehen ist, die im implantierten Zustand Gewebe oder Gefäßwände des Freisetzungsgebiets gegen den Wirkstoff (50) schützt.

12. Implantat nach einem der vorhergehenden Ansprüche, **dadurch gekennzeichnet, dass** der Trägerkörper (10) als Stent ausgebildet ist.

13. Implantat nach einem der vorhergehenden Ansprüche, **dadurch gekennzeichnet, dass** der Trägerkörper (10) als Rohr oder Stab ausgebildet ist.

14. Implantat nach einem der vorhergehenden Ansprüche, **dadurch gekennzeichnet, dass** der Trägerkörper 10 aus einer gerollten Folie (11) gebildet ist, die Vertiefungen (16) zur Aufnahme des Wirkstoffs (50) aufweist.

15. Implantat nach einem der vorhergehenden Ansprüche, **dadurch gekennzeichnet, dass** der Trägerkörper (10) aus einer gerollten und geschlitzten Folie (11) gebildet ist.
